# EUROPEAN PATENT SPECIFICATION

(11) **EP 4 114 973 B1**
(45) Date of publication and mention of the grant of the patent: **13.08.2025**
(21) Application number: 21707724.7
(22) Date of filing: 01.03.2021
(51) Int. Cl.: C12Q 1/6883, C12Q 1/6818

(54) **HYDROLYSIS-BASED PROBE AND METHOD FOR STR GENOTYPING**
HYDROLYSEBASIERTE SONDE UND VERFAHREN ZUR STR-GENOTYPISIERUNG
SONDE À BASE D'HYDROLYSE ET PROCÉDÉ DE GÉNOTYPAGE STR

(30) Priority: 03.03.2020 EP 20160679
(43) Date of publication of application: 11.01.2023
(73) Proprietor: Universiteit Gent, 9000 Gent (BE)
(72) Inventor: TYTGAT, Olivier, 9820 Merelbeke (BE); VAN NIEUWERBURGH, Filip, 9820 Merelbeke (BE); DEFORCE, Dieter, 8520 Kuurne (BE)
(86) International application number: PCT/EP2021/055000
(87) International publication number: WO 2021/175762

(56) References cited:
- WO-A1-2009/053679
- WO-A1-2013/107857
- WO-A1-2019/011971
- WO-A1-2019/162240
- WO-A1-99/43853
- US-A1- 2012 088 246
- FRENCH D J ET AL: "Interrogation of short tandem repeats using fluorescent probes and melting curve analysis: A step towards rapid DNA identity screening", FORENSIC SCIENCE INTERNATIONAL: GENETICS, ELSEVIER BV, NETHERLANDS, vol. 2, no. 4, 1 September 2008 (2008-09-01), pages 333 - 339, XP023438759, ISSN: 1872-4973, [retrieved on 20080610], DOI: 10.1016/J.FSIGEN.2008.04.007
- MICAH D. HALPERN ET AL: "An STR Melt Curve Genotyping Assay for Forensic Analysis Employing an Intercalating Dye Probe FRET* : STR FRET MELT CURVE GENOTYPING ASSAY", JOURNAL OF FORENSIC SCIENCES, vol. 56, no. 1, 14 September 2010 (2010-09-14), CHICAGO, IL, US, pages 36 - 45, XP055728133, ISSN: 0022-1198, DOI: 10.1111/j.1556-4029.2010.01549.x

## Description

### Technical field of the invention

The present invention relates to genotyping samples which contain short tandem repeats (STRs). The present invention discloses fluorescently labelled hybrid DNA:RNA probes consisting of 3 DNA regions wherein one region contains at least 1 RNA residue and another region contains at least 1 quencher. The present invention further relates to a method utilising said probes and the RNase H2 enzyme which recognises the RNA:DNA duplex that is formed when the probe hybridises to a DNA sample containing STRs. The enzyme cleaves off the region containing the quencher resulting in an increased fluorescent signal. Hybridisation, followed by enzymatic recognition and subsequent probe cleavage occurs at a higher temperature when the number of repeats in the sample corresponds exactly to the number of repeats in the probe. This present probe and method are particularly useful in a portable device for forensic DNA analysis.

### Background of the invention

Deoxyribonucleic acid (DNA) is used for identification purposes of individuals, including kinship analysis and forensic DNA genotyping. Polymorphisms in the DNA, e.g. Short Tandem Repeats (STRs) and Single Nucleotide Polymorphisms (SNPs) are examined for this goal. STRs remain the polymorphism of choice for many applications. STR-loci are characterised by short (typically 4 nucleotides) repeating sequences that are polymorphous within a certain population regarding the amount of repeats. [1]

In the human genome, different regions containing this specific type of polymorphism are identified. A statistically unique profile is obtained by analysing a large number of STR-loci, mostly located in the noncoding regions of the human genome for forensic purposes. In Europe, typically a panel of 12 STRs was examined, called the European Standard Set (ESS). This panel is now expanded with 5 additional loci. [2] In the US, the Combined DNA Index System (CODIS) is used, containing 13 core loci and 7 additional loci. [3]

Typically, these loci are analysed by means of capillary electrophoresis (CE), a DNA size separation technique. CE is a lengthy process that requires bulky equipment. Moreover, the high potential needed for the electrophoresis implicates the need for an accurate power supply. Altogether, CE is not ideally suited to be implemented in a portable device. Standalone devices, e.g. the RapidHIT (Applied Biosystems) [4] are on the market. This particular device has a mass of 82 kg, thereby hampering the on-site analysis of DNA traces in the routine.

Crime investigations would benefit tremendously from on-site DNA analysis, as this could speed up the inquiry. Besides that, implementation of these analyses on a chip would reduce the risk for contamination, avoid the need for highly trained staff and lower the cost for the society. [5]

Alternative detection methods for STR genotyping that could potentially be integrated in a portable device have been described. Almost all of them are hybridisation based approaches, using so-called STR probes. STR loci are rather long compared to SNP loci, which implicates the need for long probes. Hybridisation based methods rely on duplex stability. A partial mismatch between sample and probe, herein called hetero-duplex formation, will result in destabilisation of the duplex, reflected by a lower melting temperature. However, the longer a probe is, the lower the impact of a mismatch on duplex stability is. Not only are STR-loci by definition long, the possible alleles have a high degree of similarity, due to the presence of repeating units in the probe: even when a probe and a sample do not share the same amount of repeats, there is a large fraction of the probe that matches the sample perfectly, with only a small fraction showing a mismatch with the sample.

In order to increase the destabilising effect of a 1-repeat mismatch, US9404148B2 [6, 7] describes the HyBeacon probes that are used in solution, along with a blocker oligonucleotide, thereby shortening the probe length. This assay was implemented in the ParaDNA device, commercialised by LGC [8]. Genotyping is done by traditional melting curve analysis. Drawbacks to this system are probe design restrictions, making design of a system capable of genotyping all the loci needed for a complete DNA profile impossible, and the need for a second oligonucleotide functioning as a blocker, thereby adding a significant degree of complexity to the system. Other systems using multiple synthetic oligonucleotides are described, e.g. US9783842B [11] which describes a method based on differential hybridisation, US7501253B2 [12] which describes a branch migration assay and US6753148B2 which describes methods based on the stability of probe and sample duplex, namely a 'sandwich hybridisation method' using a capture probe and a reporter probe and a 'loop-out method' [13]. Similar drawbacks, e.g. the increase of complexity, as described for the HyBeacon probes are also encountered in the latter systems.

US12/276849 [9, 10] describes the dpFRET methodology, which is a melting curve based approach omitting the use of blocking oligos. Drawback to this system is the use of a toxic intercalating dye, which also alters the melting behaviour of oligos.

Besides the use of multiple synthetic oligonucleotides, the introduction of an enzymatic cleavage step is a valid strategy to enhance the specificity of an assay relying on duplex destabilisation dramatically. Using the dpFRET methodology or any other methodology relying on the physical distance between probe and sample, melting peaks are relatively broad as a signal is already being generated during the process of annealing. On the contrary, an endonuclease relies on the correct basepairing of DNA. A signal is therefore only generated after duplex formation, resulting in more narrow and distinctive peaks.

A suitable enzyme for genotyping assays is the RNase H2 enzyme, which recognises an RNA:DNA duplex and cleaves the RNA strand. US20160130673A1 [19] describes the combined use of endonuclease activity (e.g. originating from RNase H) and exonuclease (e.g. originating from a polymerase) for detection of a target sequence. Said system is rather similar to TaqMan^{®} probes but makes use of a chimeric DNA-RNA-DNA probe. The probe targets small regions of interest, e.g. a SNP or an INDEL and relies on whether or not the RNA-region of the probe hybridises to the target region of interest. The probes are further designed in such a way that the mismatch is positioned in the centre of the duplex, which is the most destabilizing position. Such an assay results in a binary answer (i.e. either the RNA moiety will hybridise or not) which is a characteristic ideally suited for the analysis of bi-allelic loci, e.g. SNP-loci. However, such a strategy cannot be applied for STR-probes, as these DNA regions are characterised by multiple possible alleles that differ in length rather than solely in sequence. Indeed, the sensing part of such a probe cannot be positioned in the centre of the probe, but more towards a terminus. Therefore, some structural adaptations (e.g. an anchor region and the positioning of the RNA-base) to this probe are indispensable. As the loci of interest are longer than SNP-loci, the destabilizing effect of a mismatch decreases. This implicates that the RNA-moiety will hybridize even when a mismatch occurs, complicating the method of assessment and data analysis.

Taken together, there is clearly still a high need to design an STR genotyping probe and method that results in a high signal-to-noise, has no design limitations and can be implemented in a portable device.

### Brief description of figures

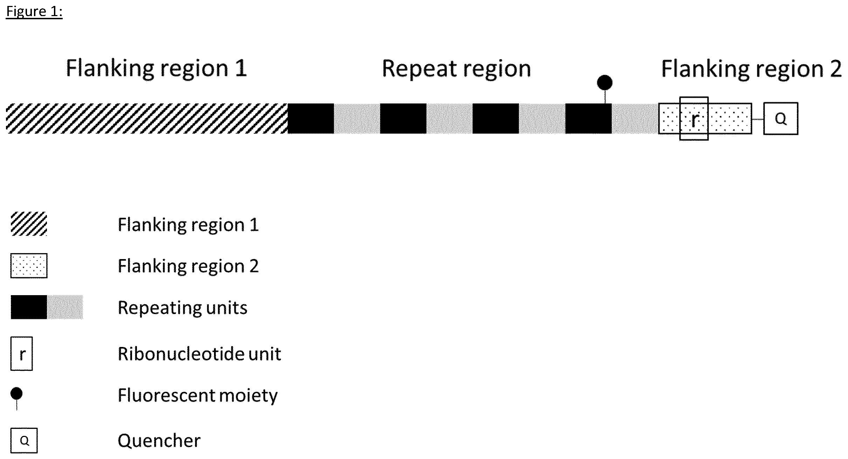

**Figure 1****:** Probe design. A probe consists, from 5' to 3' or from 3' to 5' of (i) a first flanking region, acting as an anchor in order to ensure proper annealing of the probe and preventing slippage; (ii) a repeat region, comprising one or multiple repeats and comprising at least one fluorescent moiety; (iii) a second flanking region, acting as a sensor, comprising at least one ribonucleotide and at least one quencher capable of quenching the said fluorophore.

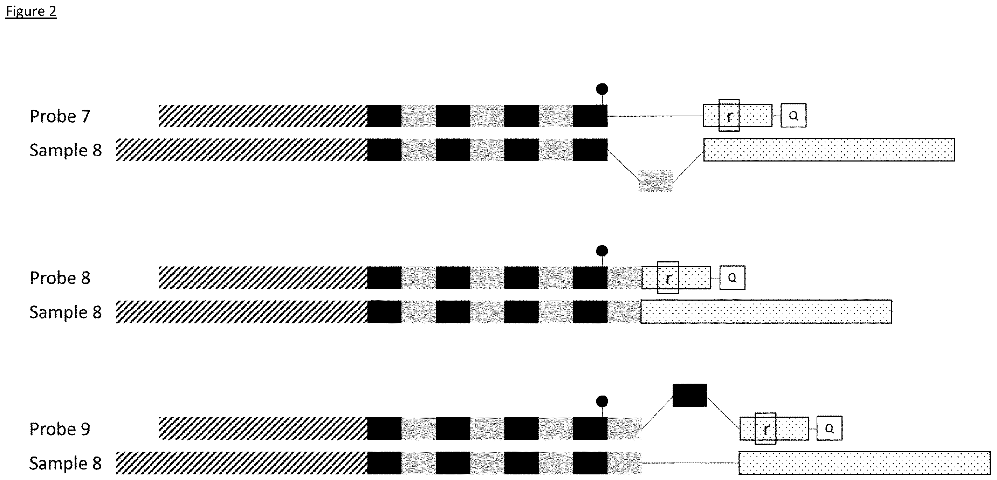

**Figure 2****:** Probe:sample (hetero)-duplexes before enzymatic digestion. If the probe and the sample have the same repeat number, indicating full complementarity, a homo-duplex is formed. Whereas, when the probe and the sample do not share the same amount of repeats, a hetero-duplex will be formed, characterised by a lower hybridisation and melting temperature.

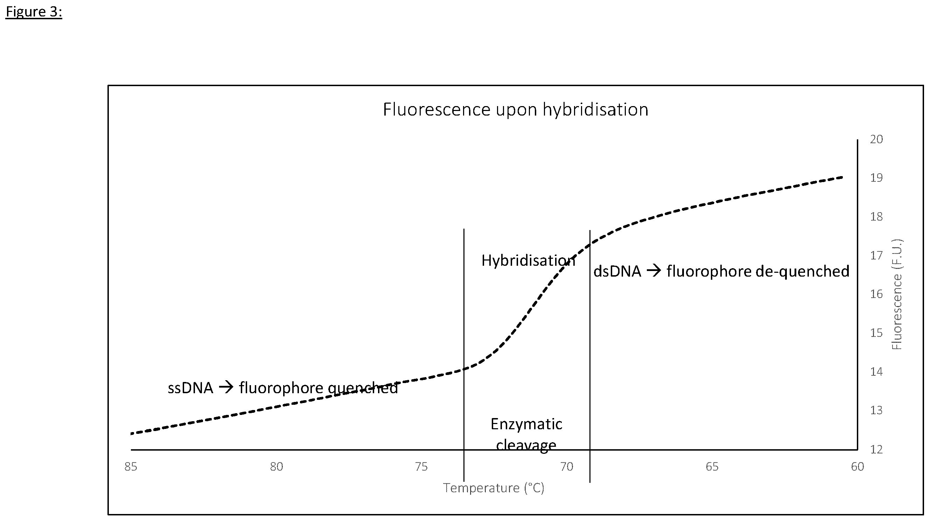

**Figure 3****:** Fluorescence upon hybridisation. At a high temperature, DNA is single stranded (denaturation) and probes remain intact. Upon cooling down, probes and sample anneal. The RNase H2 enzyme recognises and cleaves the probe at the RNA position, causing the quencher and the fluorophore to be separated from each other. That, on its turn, causes an increase of fluorescence. Mind the inversed direction of the temperature axis.

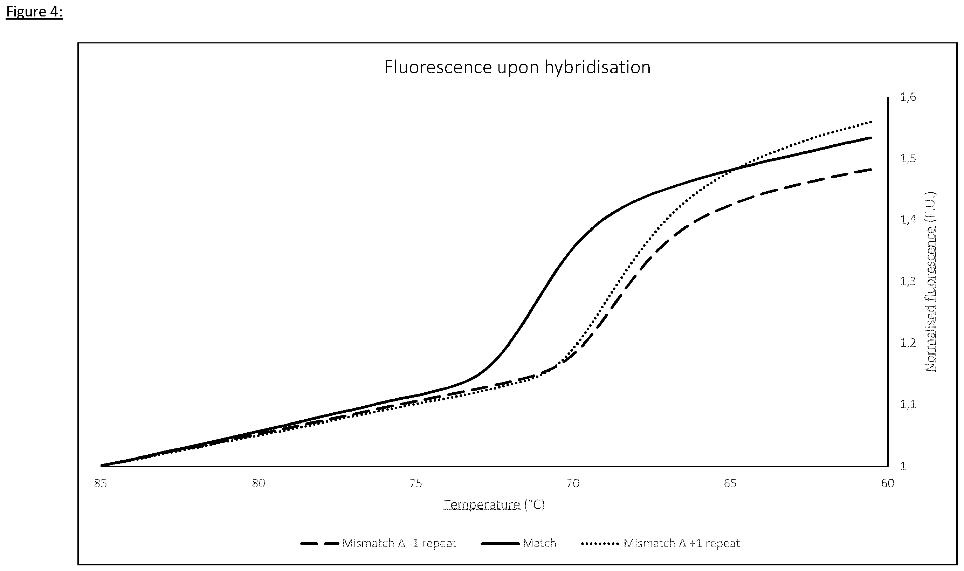

**Figure 4****:** Fluorescence upon hybridisation, 3 different situations. One sample was incubated with 3 different probes: a matching probe (full line), a probe having one repeat less as compared to the sample (dashed line) and a probe having one repeat more as compared to the sample (dotted line). An increase of fluorescence indicates hybridisation of the RNA-moiety. This occurs at the highest temperature for the matching probe, although the probe having one more repeat is longer and therefore has a theoretical higher melting temperature. Mind the inversed direction of the temperature axis.

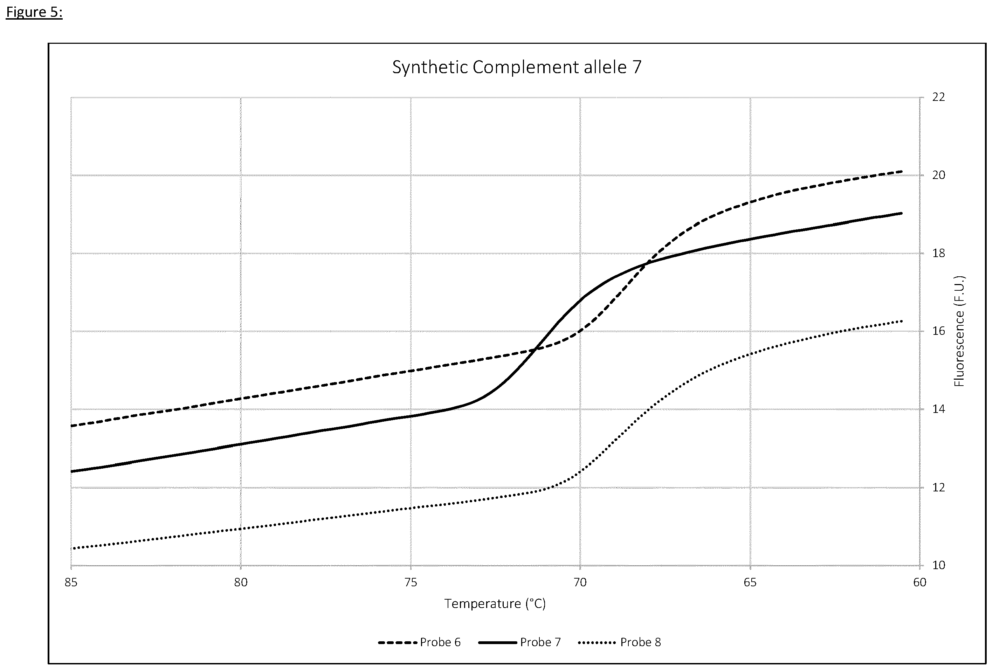

**Figure 5****:** Fluorescence as a function of temperature, example 1. Matching probe 7 hybridises at a higher temperature as compared to the mismatch probes 6 and 8.

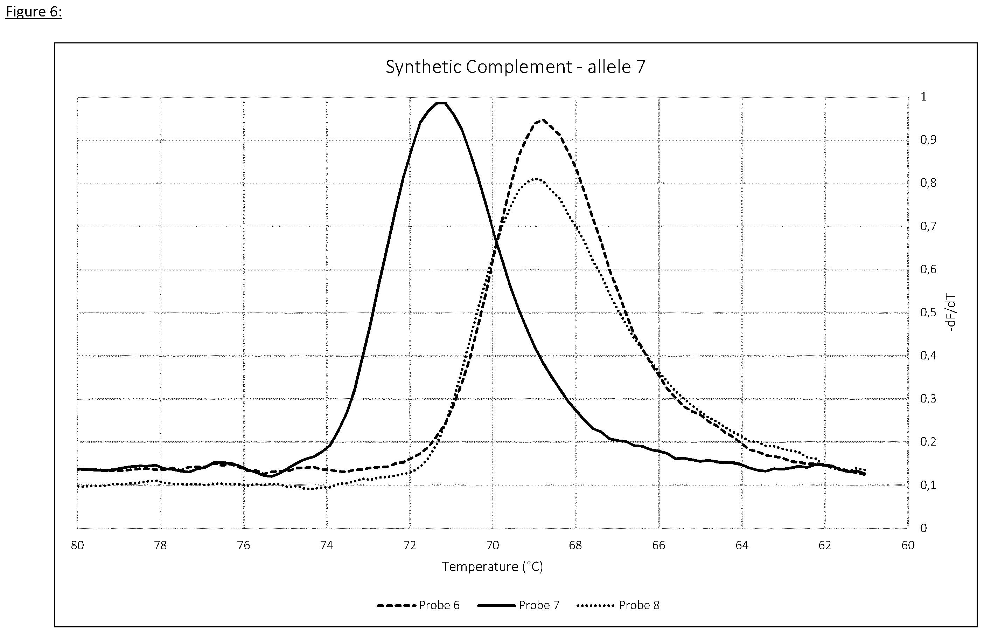

**Figure 6****:** First derivative of fluorescence with respect to temperature, example 1. Matching probe 7 hybridises at a higher temperature as compared to the mismatch probes 6 and 8.

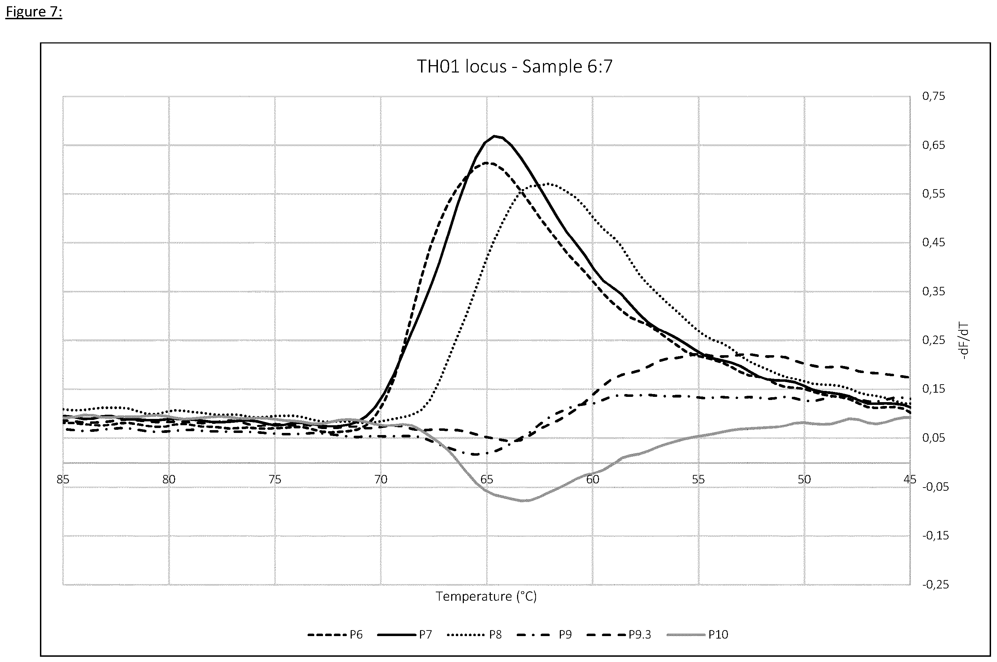

**Figure 7****:** First derivative of fluorescence with respect to temperature, example 2. Matching probes 6 and 7 hybridise at a higher temperature as compared to mismatch probe 8. No signal occurs for mismatch probes 9, 9.3 and 10.

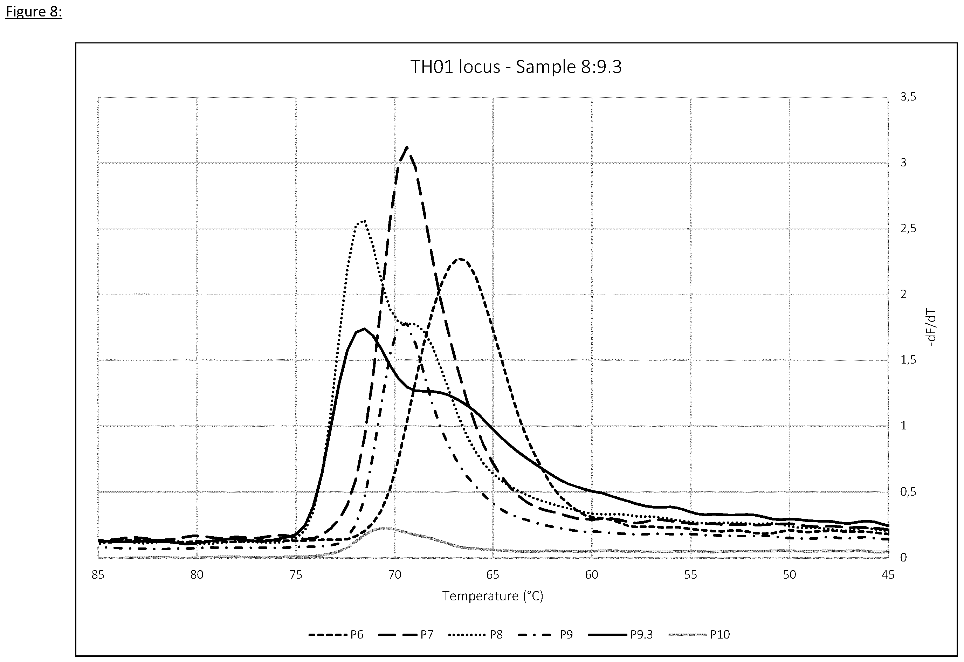

**Figure 8****:** First derivative of fluorescence with respect to temperature, example 3. Matching probes 8 and 9.3 hybridise at a higher temperature as compared to mismatch probe 6, 7 and 10. Only a very limited signal occurs for mismatch probe 10.

### Summary of the invention

The present invention relates to a composition comprising:
a) an array of oligonucleotide probes wherein each of said probes comprises, from 5' to 3' or from 3' to 5, the following 3 regions:
   I. a first flanking region comprising at least one nucleotide, which anneals with a region directly next to the specific DNA sequence of interest and which has a higher melting temperature than the second flanking region,
   II. a region comprising a specific DNA sequence which anneals with the short tandem repeat region of interest within a sample and which contains at least one fluorophore, and
   III. a second flanking region comprising at least 2 nucleotides, and which contains at least one ribonucleotide and at least one quencher moiety capable of efficiently quenching said fluorophore, wherein the fluorophore and the quencher moiety are separated from each other by at least one ribonucleotide, and
b) the RNase H2 enzyme capable of digesting said probe by recognition of the RNA:DNA duplex upon hybridization of said probe with said sample.

The present invention further relates to a composition comprising an array of oligonucleotide probes as described above wherein said quencher is attached to the 3' or 5' terminus of each of said probes.

The present invention further relates to a composition comprising an array of oligonucleotide probes as described above wherein the said fluorophore is attached to a nucleotide of the second flanking region of each of said probes and wherein the said quencher is attached to a nucleotide of the specific DNA sequence of interest of each of said probes.

In a specific embodiment of this invention, the said fluorophore is a fluorescein derivate.

In a specific embodiment of this invention, the said quencher is an Iowa Black FQ quencher.

The present invention further relates to a composition comprising an array of oligonucleotide probes as described above containing more than one ribonucleotide.

The present invention further relates to a composition comprising an array of oligonucleotide probes as described above wherein said nucleotides are nucleic acid analogues.

The present invention further relates to a composition comprising an array of oligonucleotide probes as described above wherein each of said probes is immobilised on a support.

The present invention also relates to a method to genotype short tandem repeats within a sample comprising the steps of:
- providing a sample comprising DNA,
- amplifying DNA within said sample which comprises a specific DNA sequence of interest in order to obtain amplified single stranded DNA sequences,
- adding an array of probes as described above to said DNA sequences to obtain duplexes of single stranded DNA sequences annealed to said probes,
- adding RNase H2 enzyme,
- heating the mixture of sample, probe and RNase H2 enzyme to a temperature at which the RNase H2 enzyme is activated,
- measuring fluorescence upon cooling down the said mixture after activation of the RNase H2 enzyme wherein the increase of fluorescence intensity provides information on whether or not a specific, completely complementary short tandem repeat is present in said sample.

The present invention further relates to a method to genotype as described above wherein said amplification within said sample is undertaken by an asymmetric PCR in order to obtain amplified, single stranded DNA sequences.

The present invention further relates to a method to genotype as described above wherein said amplification within said sample is undertaken by a symmetric PCR using biotin-labelled primers or a subsequent lambda exonuclease digestion in order to obtain amplified, single stranded DNA sequences.

The present invention further relates to a method to genotype as described above wherein said array of probes is added in solution, or is immobilised onto a support.

### Description of the invention

The present invention relates to a composition comprising an array of oligonucleotide probes and the RNase H2 enzyme. A probe is herein defined as a synthetically manufactured oligonucleotide, consisting of 2 or more nucleotides and/or ribonucleotides covalently linked to each other, wherein some nucleotides and/or ribonucleotides might be modified. Such a modification is defined as a molecule attached to the oligonucleotide that not necessarily occurs in natural DNA or RNA. Examples of modifications are e.g. the presence of a fluorescent moiety, the presence of a quencher, the presence of molecules for attachment purposes, modifiers of the melting temperature etc. Probes can be synthetically manufactured, but the definition of an oligonucleotide probe is herein not narrowed down to exclusively synthetically manufactured oligonucleotides. Probes are generally designed in such a way that they will interact with the investigated molecule, and the response of the probe upon this interaction will be observed and used in order to obtain information of the said investigated molecule.

DNA complementarity can be explained by Chargaff's rules, stating that adenine always forms hydrogen bonds with thymine or uracil, and cytosine with guanine, a process also referred to as Watson-Crick or Hoogsteen base pairing, resulting in double stranded DNA. Hybridisation or annealing is defined herein as the formation of a duplex or hetero-duplex structure, consisting of two nucleic acid strands after complementary base pairing. A duplex structure is defined as a complex of 2 fully complementary base paired nucleic strands. A hetero-duplex structure is defined as a complex of 2 partially complementary nucleic acid strands, e.g. 2 DNA strands deferring by one or more 4-nucleotide repeats.

The function of the array of probes disclosed by this invention is genotyping of Short Tandem Repeat-loci (STR-loci). STR-loci are characterised by short (typically 4 nucleotides) repeating sequences that are polymorphous within a certain population regarding the amount of repeats. In contrast to Single Nucleotide Polymorphisms (SNPs), these loci are multi-allelic, indicating that a rather broad range of repeat numbers occur within the population. By determining the repeat number of sufficient loci, a statistically unique profile is obtained for an individual. The wording 'array of probes' refers to the fact that for each allele of the investigated STR-locus, a dedicated probe is designed. The array of probes consists of all different probes for a certain locus. The interaction between a specific probe and a sample should be analysed separately, implicating that all different probes should be physically separated, for example by means of different wells on a multi-well plate, or by immobilizing them in distinct spots on a surface.

The oligonucleotide probes disclosed by this invention comprise, from 5' to 3' or from 3' to 5' of a first flanking region, a specific DNA sequence of interest and a second flanking region, as illustrated in figure 1.

The first flanking region is a sequence of nucleotides and comprises at least one nucleotide. In a more convenient embodiment of this invention, the flanking region comprises between 20 and 40 nucleotides. The first flanking region is complementary to and anneals with the region directly next to the STR-region and ensures proper annealing of the sample and the probe, therefore acting as an anchor. As this first flanking region has a pronounced higher melting temperature as compared to the second flanking region, initiation of the hybridisation is privileged at the first flanking region. In order to obtain correct genotyping, it is crucial that the first repeat of the sample anneals to the first repeat of the probe, and slippage of the sample is prevented.

The specific DNA sequence of interest comprises at least 1 short tandem repeat and contains at least one fluorophore and anneals with the short tandem repeat region within the sample. In one embodiment of this invention, this sample is DNA where the target STR-regions are amplified by means of e.g. polymerase chain reaction.

The second flanking region comprises at least 1 nucleotide and contains at least one ribonucleotide, e.g. ATP, CTP, GTP and UTP and contains at least one quencher moiety capable of efficiently quenching the said fluorophore.

Fluorophores are herein defined as compounds characterised with a fluorescent emission maximum between about 350 nm and 900 nm. A commonly used fluorescein derivate is 5-FAM (5-carboxyfluorescein). Other commonly used fluorophores are 5-Hexachloro-Fluorescein, 6-Hexachloro-Fluorescein, 5-Tetrachloro-Fluorescein5-TAMRA (5-carboxytetramethylrhodamine), 6-TAMRA (6-carboxytetramethylrhodamine), Cy5 (Indodicarbocyanine-5); Cy3 (Indo-dicarbocyanine-3), and BODIPY FL (2,6-dibromo-4,4-difluoro-5,7-dimethyl-4-bora-3a,4a-diaza-s-indacene-3-proprionic acid).

A quencher is defined as a moiety that suppresses luminescence of a fluorophore moiety when brought into proximity of said fluorophore. A common mechanism of quenching is fluorescence resonance energy transfer (FRET), but the definition of a quencher is herein not narrowed down to this mechanism. Other mechanisms are e.g. photo-induced electron transfer. Commercially available quenchers are: Dabcyl, Iowa Black^{®} FQ and RQ, ZEN^{™} and Black Hole quenchers, e.g. BHQ-1^{®}.

In a specific embodiment of this invention, a fluorescein derivate is used in combination with an Iowa Black FQ quencher moiety. Those skilled in the art will recognise that other combinations of fluorescent moieties and quenchers are suitable for this goal. It is crucial that the emission wavelength of the fluorophore corresponds to the optimal absorbance wavelength of the quencher. An example of a possible combination of fluorophore and quencher is Cy3 with Black Hole Quencher 2.

The fluorophore and the quencher moiety are separated from each other by at least one ribonucleotide. If the quencher and the fluorescent moiety were to be linked to the same nucleotide or ribonucleotide, no signal would occur upon digestion of the probe by a suitable enzyme, as both said moieties would not be separated from each other. In a more specific embodiment of this invention, the fluorophore and the quencher are separated by 15 to 30 nucleotides.

In a specific embodiment of this invention, the quencher is attached to the 3' or 5' terminus of the probe.

The present invention further relates to oligonucleotide probes as described above wherein the said fluorophore is attached to a nucleotide of the second flanking region and wherein the said quencher is attached to a nucleotide of the specific DNA sequence of interest. The present invention further relates to oligonucleotide probes as described above which comprises more than one ribonucleotide.

The present invention further relates to oligonucleotide probes as described above wherein said nucleotides are nucleic acid analogues, e.g. LNA, PNA, GNA, TNA, morpholino (PMO).

The said probes described in this invention are functional both in solution and immobilised on a support.

The present invention also relates to a method to genotype short tandem repeats within a sample comprising the steps of:
1. Providing a sample comprising DNA. In a more specific embodiment of this invention, the sample comprises DNA with at least one STR-locus. The source of this DNA can be human, animal, or even plants. Those skilled in the art will recognise this is not a limitative list.
2. Amplifying DNA within said sample which comprises a specific DNA sequence of interest in order to obtain amplified DNA sequences. There are multiple strategies to amplify DNA, however, the polymerase chain reaction (PCR) is the most commonly applied method used in order to amplify specific sequences of interest, e.g. STR-loci. In a PCR-reaction, the amplified loci are determined by specifically designed primers. Amplification is performed using a DNA polymerase enzyme. Those skilled in the art will recognise the existence of many other strategies to amplify DNA, both targeted or untargeted. Examples are isothermal DNA amplification, whole genome amplification, and rolling circle amplification.
3. Adding a probe as described above to said amplified DNA sequences to obtain duplexes of single stranded DNA sequences annealed to said probe.
4. Adding RNase H2 enzyme or any other enzyme capable of cleaving the probe at the RNA-position when this position is hybridised to a complementary nucleotide. In a specific embodiment of this invention, 25 mU of the enzyme is added.
5. Heating the mixture of sample, probe and RNase H2 enzyme to a temperature at which the RNase H2 enzyme is activated, typically 95°C, using e.g. a real-time PCR instrument.
6. Measuring fluorescence upon cooling down the said mixture after activation of the RNase H2 enzyme using e.g. a real-time PCR instrument.

After activation of the RNase enzyme at a high temperature, the mixture is cooled down slowly. In a specific embodiment of this invention, the mixture is cooled down at a rate of 0.5°C per minute. However, it should be noted that both faster and slower cooling is feasible. Upon cooling down of the mixture, the probes will hybridise with the amplified DNA strands of the sample. Owing to the presence of an anchor region in the probe, hybridisation is privileged at the anchor-side of the probe. This ensures that the first repeat of the probe, starting from the anchor-side, will hybridise to the first repeat of the sample.

If said probe and the amplified DNA-strand have the exact same number of repeats, the complete probe will hybridise to the sample. When the probe and the amplified DNA-strand do not share the same repeat number, hetero-duplex formation will occur (figure 2). In the latter situation, hybridisation will occur at a lower temperature as compared to the situation of full complementarity. As the RNase H2 enzyme is active in a broad range of temperatures, the probe is cleaved as soon as it hybridises to the sample (figure 3). As a consequence, the fluorescent signal of probes with the same number of repeats as the sample increases at a higher temperature as compared to mismatch probes (figure 4).

The present invention thus describes an STR-assay determining the hybridisation temperature of a probe by enzymatic digestion. The destabilizing effect of a partial mismatch between probe and sample is hard to assess for STR-loci, as these are by definition long loci. It is generally known that the longer a probe is, the lower the destabilizing effect of a mismatch is. By introducing an enzymatic cleavage step that relies on the specific hybridisation of an RNA unit in the probe, extremely sharp and distinct peaks are obtained, thereby optimally highlighting the difference in duplex stability. Only after specific hybridisation of this ribonucleotide, implying the formation of an open loop structure in the hetero-duplex (as illustrated in figure 2), a signal is generated. The use of a fluorescent molecule in combination with a quencher moiety results in a high signal-to-noise ratio.

The present invention further relates to a method to genotype as described above wherein said amplification within said sample is undertaken by an asymmetric PCR in order to obtain amplified, single stranded DNA sequences. Obtaining single stranded DNA is crucial, as re-annealing of double stranded amplicons would be favoured above probe hybridisation. Asymmetric PCR is an often used technique to obtain single stranded DNA. In order to obtain this goal, the primers are added to the PCR reaction mixture in a different concentration. The primer that will be incorporated in the strand complementary to the probe will be added in excess. During the first PCR cycles, both primers will be consumed and PCR will occur exponentially. Upon depletion of the primer added in a lower concentration, PCR will occur linearly, as only the desired strand is produced.

An alternative for asymmetric PCR is symmetric PCR using a biotin-labelled primer. After PCR, the streptavidine beads are added to the amplified DNA. The biotin labelled primers will react covalently with the streptavidine, upon denaturation of the double stranded amplicons, the desired strand can be isolated. Another alternative is symmetric PCR with subsequent lambda exonuclease digestion. Only strands originating from a 5' phosphate labelled primer will be digested.

The present invention also relates to a method as described above wherein said probes are added in solution, or are immobilised onto a support.

### Examples

### Example 1:

3 different probes (having 6, 7 and 8 repeats) designed for the TH01 locus were mixed with a synthetically manufactured complement that has 7 repeats. Concentration of the probes was 0.1 µM, concentration of the synthetic complement was 1 µM. Probe sequences can be found in table 1. After adding RNase H2 enzyme, the mixture was heated to 95°C for 10 minutes. Hereafter, the mixture was slowly cooled down in order to ensure proper hybridisation of the probes and the synthetic complement. During this hybridisation phase, fluorescence was monitored. The first derivative of the fluorescence with respect to the temperature was calculated.

**Table 1: Sequence of oligonucleotides used for the TH01 experiment. Ribonucleotides are preceded by 'r'. An underlined T-nucleotide indicates fluorescein dT. Iowa Black FQ was used as the quencher.**

| **Name** | **Sequence** |
|---|---|
| TH01 Probe 6 | 5'CTGTTCCTCCCTTATTTCCCTCATTCATTCATTCATTCATTCATTCACCrATG-Iowa Black FQ (SEQ ID N° 1) |
| TH01 Probe 7 | 5'CTGTTCCTCCCTTATTTCCCTCATTCATTCATTCATTCATTCATTCATTCACCrATG- Iowa Black FQ (SEQ ID N° 2) |
| TH01 Probe 8 | 5'CTGTTCCTCCCTTATTTCCCTCATTCATTCATTCATTCATTCATTCATTCATTCACCrAT G-Iowa Black FQ (SEQ ID N° 3) |
| TH01 Synthetic complement 6 | 5'ACAGACTCCATGGTGAATGAATGAATGAATGAATGAATGAGGGAAATAAGGGAGG AACAGGCCAATGGGAATCAC (SEQ ID N° 4) |
| TH01 Synthetic complement 7 | 5’ACAGACTCCATGGTGAATGAATGAATGAATGAATGAATGAATGAGGGAAATAAGG GAGGAACAGGCCAATGGGAATCAC (SEQ ID N° 5) |
| TH01 Synthetic complement 8 | 5'ACAGACTCCATGGTGAATGAATGAATGAATGAATGAATGAATGAATGAGGGAAAT AAGGGAGGAACAGGCCAATGGGAATCAC (SEQ ID N° 6) |

Fluorescence dropped in all 3 wells, indicating that all different probes were digested by the enzyme. However, the matching probe was digested at a higher temperature as compared to the mismatch probes. This indicates hetero-duplex formation of the mismatch probes and the sample.

### Example 2:

A buccal swab was immersed in a volume of 200 µL sterile HPLC-water. After a vortex-step of 30", the swab was removed and the water was used as input for PCR. Singleplex asymmetric PCR was performed with 30 µL of input sample. Primer concentrations were 0.1 µM forward primer and 1.5 µM reverse primer. The volume of the PCR mixture was 50 µL, containing MgCl²⁺ at a concentration of 0.5 mM, dNTP's at 200 µM each, 1X Qiagen PCR buffer and 1.3 U HotStarTaq enzyme. Activation of the polymerase was done by heating the PCR mix at 95°C for 15 minutes followed by 60 cycles of 95°C for 1 minute, 59°C for 1 minute and 72°C for 80 seconds. Primer sequences can be found in table 1.

After asymmetric PCR, aliquots of 8.5 µL amplified product were divided in a 96-Well plate. To each separate well, 1.5 µL of one particular probe was added at a starting concentration of 1 µM. These mixtures were denatured for 10 minutes at 95°C, followed by slowly cooling at a ramp rate of 0.04 °C/s while fluorescence was continually measured using a LightCycler (Roche). The first derivative of the hybridisation curve is calculated, resulting in melting peaks. Probe sequences can be found in table 2. The sample was genotyped using CE-analysis and had alleles 6 and 7.

**Table 2: Sequence of oligonucleotides used for the TH01 experiment. 'r' denotes the following unit is a ribonucleotide. An underlined T-nucleotide indicates fluorescein dT. Iowa Black FQ was used as quencher.**

| **Name** | **Sequence** |
|---|---|
| TH01 Forward primer | 5'GTGATTCCCATTGGCCTGTTC (SEQ ID N° 7) |
| TH01Reverse primer | 5'ATTCCTGTGGGCTGAAAAGCTC (SEQ ID N° 8) |
| TH01 Probe 6 | 5'CTGTTCCTCCCTTATTTCCCTCATTCATTCATTCATTCATTCATTCACCrATG-Iowa Black FQ (SEQ ID N° 9) |
| TH01 Probe 7 | 5'CTGTTCCTCCCTTATTTCCCTCATTCATTCATTCATTCATTCATTCATTCACCrATG- Iowa Black FQ (SEQ ID N° 10) |
| TH01 Probe 8 | 5'CTGTTCCTCCCTTATTTCCCTCATTCATTCATTCATTCATTCATTCATTCATTCACCrAT G-Iowa Black FQ (SEQ ID N° 11) |
| TH01 Probe 9 | 5'CTGTTCCTCCCTTATTTCCCTCATTCATTCATTCATTCATTCATTCATTCATTCACCrAT G-Iowa Black FQ (SEQ ID N° 12) |
| TH01 Probe 9.3 | 5'CTGTTCCTCCCTTATTTCCCTCATTCATTCATCATTCATTCATTCATTCATTCATTCATT CACCrATG-Iowa Black FQ (SEQ ID N° 13) |
| TH01 Probe 10 | 5'CTGTTCCTCCCTTATTTCCCTCATTCATTCATTCATTCATTCATTCATTCATTCATTCAC CrATG-Iowa Black FQ (SEQ ID N° 14) |

The first derivative of all hybridisation curves is shown in figure 7. A pronounced signal can be observed for alleles 6, 7 and 8. Although probe 8 is the longest probe of the 3 probes displaying a signal, it shows a distinctly lower hybridisation temperature, indicating hetero-duplex formation. The other probes show barely any signal, indicating that the mismatch was too destabilising for hybridisation to occur.

### Example 3:

A buccal swab was prepared, amplified and analysed as described in example 2. The same primers and probes were used as described in example 2. The examined sample was genotyped using CE having allele 9.3. Samples with allele 9.3 have 10 repeats but are characterised by a deletion of 1 nucleotide in their 3^{rd} repeat. These are challenging alleles as hybridisation of this sample with probe 10 results in a hetero-duplex only being destabilised by a one-nucleotide indel (insertion/deletion).

After asymmetric PCR, aliquots of 8.5 µL amplified product were divided in a 96-Well plate. To each separate well, 1.5 µL of probe (1µM) was added. These mixtures were denatured for 10 minutes at 95°C, followed by slowly cooling at a ramp rate of 0.04 °C/s while fluorescence was continually measured using a LightCycler (Roche). The first derivative of the hybridisation curve is calculated, resulting in melting peaks.

The first derivative of all hybridisation curves is shown in figure 8. A pronounced signal can be observed for alleles 8 and 9.3. Probe 10, having only a 1-nucleotide mismatch with the positive allele 9.3, shows barely no signal. Probe 9, being a neighbouring probe for both positive alleles, and probe 7, also a neighbouring probe for a positive allele, show melting peaks at a lower temperature as compared to the positive probes. The positive probes display a second peak at a lower temperature, which can be addressed to the formation of a hetero-duplexes between probe 8 and sample 9.3, and vice versa.

### References

1. Butler, J.M., Forensic DNA typing: biology, technology, and genetics of STR markers. 2005: Elsevier.
2. Schneider, P.M., Expansion of the Europian standard set of DNA database loci-the current situation. Profiles in DNA, 2009. 12: p. 6-7.
3. Hares, D.R., Selection and implementation of expanded CODIS core loci in the United States. Forensic Science International: Genetics, 2015. 17: p. 33-34.
4. Hennessy, L.K., et al., Developmental validation studies on the RapidHIT™ human DNA identification system. Forensic Science International: Genetics Supplement Series, 2013. 4(1): p. e7-e8.
5. Bruijns, B., et al., Microfluidic devices for forensic DNA analysis: A review. Biosensors, 2016. 6(3): p. 41.
6. Gale, N., et al., Oligonucleotides and uses thereof. 2016, Google Patents.
7. Gale, N., et al., Rapid typing of STRs in the human genome by HyBeacon® melting. Organic & biomolecular chemistry, 2008. 6(24): p. 4553-4559.
8. Blackman, S., et al., Developmental validation of the ParaDNA® Intelligence System-A novel approach to DNA profiling. Forensic Science International: Genetics, 2015. 17: p. 137-148.
9. Halpern, M. and P.M. Ellis, Dye probe fluorescence resonance energy transfer genotyping. 2010, Google Patents.
10. Halpern, M.D. and J. Ballantyne, An STR melt curve genotyping assay for forensic analysis employing an intercalating dye probe FRET. Journal of forensic sciences, 2011. 56(1): p. 36-45.
11. Mamone, J., M.N. Feiglin, and H. Gamper, STR genotyping by differential hybridization. 2017, Google Patents.
12. Pourmand, N., R.W. Davis, and S.X. Wang, DNA fingerprinting using a branch migration assay. 2009, Google Patents.
13. Sosnowski, R.G. and E. Tu, Methods and apparatus for detecting variants utilizing base stacking. 2004, Google Patents.
14. Moghaddam, P.P. and F.J. Herrmann, Randomized full-waveform inversion: a dimenstionality-reduction approach, in SEG Technical Program Expanded Abstracts 2010. 2010, Society of Exploration Geophysicists. p. 977-982.
15. Tsuchiya, T., Method of detecting mismatching regions. 2007, Google Patents.
16. Kemp, J., et al., DNA profiling and SNP detection utilizing microarrays. 2006, Google Patents.
17. Walder, J.A., et al., RNase H-based assays utilizing modified RNA monomers. 2014, Google Patents.
18. Walder, J.A., J. Dobosy, and M.A. Behlke, Cleavable hairpin primers. 2018, Google Patents.
19. Li, J., et al., Nucleic acid detection by oligonucleotide probes cleaved by both exonuclease and endonuclease. 2016, Google Patents.

## Claims

1. A composition comprising:
a) an array of oligonucleotide probes wherein each of said probes comprises, from 5' to 3' or from 3' to 5, the following 3 regions:
I. a first flanking region comprising at least one nucleotide, which anneals with a region directly next to the specific DNA sequence of interest and which has a higher melting temperature than the second flanking region,
II. a region comprising a specific DNA sequence which anneals with the short tandem repeat region of interest within a sample and which contains at least one fluorophore, and
III. a second flanking region comprising at least 2 nucleotides, and which contains at least one ribonucleotide and at least one quencher moiety capable of efficiently quenching said fluorophore, wherein the fluorophore and the quencher moiety are separated from each other by at least one ribonucleotide, and
b) the RNase H2 enzyme capable of digesting said probe by recognition of the RNA:DNA duplex upon hybridization of said probe with said sample.

2. A composition comprising an array of oligonucleotide probes according to claim 1 wherein said quencher is attached to the 3' or 5' terminus of each of said probes.

3. A composition comprising an array of oligonucleotide probes according to claims 1-2 wherein the said fluorophore is attached to a nucleotide of the second flanking region of each of said probes and wherein the said quencher is attached to a nucleotide of the specific DNA sequence of interest of each of said probes.

4. A composition comprising an array of oligonucleotide probes according to claims 1-3 wherein the said fluorophore is a fluorescein derivate.

5. A composition comprising an array of oligonucleotide probes according to claims 1-4 wherein the said quencher is a Iowa Black FQ quencher.

6. A composition comprising an array of oligonucleotide probes according to claims 1-5 wherein each of said probes contains more than one ribonucleotide.

7. A composition comprising an array of oligonucleotide probes according to claims 1-6 wherein said nucleotides are nucleic acid analogues.

8. A composition comprising an array of oligonucleotide probes according to claims 1-7 wherein each of said probes is immobilised on a support.

9. A method to genotype short tandem repeats within a sample comprising the steps of:
- providing a sample comprising DNA,
- amplifying DNA within said sample which comprises a specific DNA sequence of interest in order to obtain amplified single stranded DNA sequences,
- adding an array of probes according to claims 1-8 to said DNA sequences to obtain duplexes of single stranded DNA sequences annealed to said probes,
- adding RNase H2 enzyme,
- heating the mixture of sample, probe and RNase H2 enzyme to a temperature at which the RNase H2 enzyme is activated,
- measuring fluorescence upon cooling down the said mixture after activation of the RNase H2 enzyme wherein the increase of fluorescence intensity provides information on whether or not a specific, completely complementary short tandem repeat is present in said sample.

10. A method to genotype according to claim 9 wherein said amplification within said sample is undertaken by an asymmetric PCR in order to obtain amplified, single stranded DNA sequences.

11. A method to genotype according to claims 9-10 wherein said amplification within said sample is undertaken by a symmetric PCR using biotin-labelled primers or a subsequent lambda exonuclease digestion in order to obtain amplified, single stranded DNA sequences.

12. A method to genotype according to claims 9-11 wherein each of said probes are added in solution, or are immobilised onto a support.

## Patentansprüche

1. Zusammensetzung, umfassend:
a) ein Oligonukleotidsondenarray, wobei die Sonden jeweils von 5' zu 3' oder von 3' zu 5' die folgenden 3 Regionen umfassen:
I. eine erste flankierende Region, die mindestens ein Nukleotid umfasst, das beim Annealing eine Region direkt neben der spezifischen DNA-Sequenz von Interesse bindet und eine höhere Schmelztemperatur als die zweite flankierende Region aufweist,
II. eine Region, die eine spezifische DNA-Sequenz umfasst, die beim Annealing die Short-Tandem-Repeat-Region von Interesse innerhalb einer Probe bindet und mindestens ein Fluorophor enthält und
III. eine zweite flankierende Region, die mindestens 2 Nukleotide umfasst und mindestens ein Ribonukleotid und mindestens eine Quenchergruppierung, die in der Lage ist den Fluorophor zu quenchen, enthält, wobei das Fluorophor und die Quenchergruppierung durch mindestens ein Ribonukleotid voneinander getrennt sind und
b) das RNase-H2-Enzym, das in der Lage ist, die Sonde mittels Erkennung des RNA-DNA-Duplexes nach Hybridisierung der Sonde mit der Probe zu verdauen.

2. Zusammensetzung, umfassend ein Oligonukleotidsondenarray nach Anspruch 1, wobei der Quencher jeweils an den 3'- oder 5'-Terminus der Sonden angeheftet ist.

3. Zusammensetzung, umfassend ein Oligonukleotidsondenarray nach Ansprüchen 1-2, wobei das Fluorophor jeweils an ein Nukleotid der zweiten flankierenden Region der Sonden angeheftet ist und wobei der Quencher jeweils an ein Nukleotid der spezifischen DNA-Sequenz von Interesse der Sonden angeheftet ist.

4. Zusammensetzung, umfassend ein Oligonukleotidsondenarray nach Ansprüchen 1-3, wobei es sich bei dem Fluorophor um ein Fluoreszinderivat handelt.

5. Zusammensetzung, umfassend ein Oligonukleotidsondenarray nach Ansprüchen 1-4, wobei es sich bei dem Quencher um einen Iowa-Black-FQ-Quencher handelt.

6. Zusammensetzung, umfassend ein Oligonukleotidsondenarray nach Ansprüchen 1-5, wobei die Sonden jeweils mehr als ein Ribonukleotid enthalten.

7. Zusammensetzung, umfassend ein Oligonukleotidsondenarray nach Ansprüchen 1-6, wobei es sich bei den Nukleotiden um Nukleinsäureanaloge handelt.

8. Zusammensetzung, umfassend ein Oligonukleotidsondenarray nach Ansprüchen 1-7, wobei die Sonden jeweils auf einen Träger immobilisiert werden.

9. Verfahren zum Genotypisieren von Short-Tandem-Repeats innerhalb einer Probe, umfassend die Schritte des:
- Bereitstellens einer DNA umfassenden Probe,
- Amplifizierens von DNA innerhalb der Probe, die eine spezifische DNA-Sequenz von Interesse umfasst, zum Erhalt amplifizierter Einzelstrang-DNA-Sequenzen,
- Zugebens eines Sondenarrays nach Ansprüchen 1-8 zu den DNA-Sequenzen zum Erhalt von beim Annealing an die Sonden gebundenen Duplexen von Einzelstrang-DNA-Sequenzen,
- Zugebens von RNase-H2-Enzym,
- Erhitzens der Mischung von Probe, Sonde und RNase-H2-Enzym auf eine Temperatur, bei der das RNase-H2-Enzym aktiviert wird,
- Messens von Fluoreszenz direkt nach dem Abkühlen der Mischung nach Aktivierung von dem RNase-H2-Enzym, wobei die Erhöhung der Fluoreszenzintensität Informationen darüber, ob ein spezifisches vollständig komplementäres Short-Tandem-Repeat in der Probe vorhanden ist oder nicht, liefert.

10. Verfahren zum Genotypisieren nach Anspruch 9, wobei die Amplifikation innerhalb der Probe durch eine asymmetrische PCR zum Erhalten amplifizierter Einzelstrang-DNA-Sequenzen durchgeführt wird.

11. Verfahren zum Genotypisieren nach Ansprüchen 9-10, wobei die Amplifikation innerhalb der Probe durch eine symmetrische PCR unter Verwendung von Biotin-markierten Primern oder einer anschließenden lambda-Exonukleaseverdauung zum Erhalten amplifizierter Einzelstrang-DNA-Sequenzen durchgeführt wird.

12. Verfahren zum Genotypisieren nach Ansprüchen 9-11, wobei die Sonden jeweils in Lösung gegeben oder auf einen Träger immobilisiert werden.

## Revendications

1. Composition comprenant :
a) un réseau de sondes oligonucléotidiques, dans laquelle chacune desdites sondes comprend, de 5' à 3' ou de 3' à 5, les 3 régions suivantes :
I. une première région flanquante comprenant au moins un nucléotide, qui s'annèle avec une région directement voisine de la séquence d'ADN spécifique d'intérêt et qui présente une température de fusion plus élevée que la seconde région flanquante,
II. une région comprenant une séquence d'ADN spécifique qui s'annèle avec la région de répétition en tandem courte d'intérêt dans un échantillon et qui contient au moins un fluorophore, et
III. une seconde région flanquante comprenant au moins 2 nucléotides, et qui contient au moins un ribonucléotide et au moins une fraction agent d'extinction capable d'une extinction efficace dudit fluorophore, dans laquelle le fluorophore et la fraction agent d'extinction sont séparés l'un de l'autre par au moins un ribonucléotide, et
b) l'enzyme RNase H2 capable de digérer ladite sonde par reconnaissance du duplex ARN:ADN lors de l'hybridation de ladite sonde avec ledit échantillon.

2. Composition comprenant un réseau de sondes oligonucléotidiques selon la revendication 1, dans laquelle ledit agent d'extinction est fixé à l'extrémité 3' ou 5' de chacune desdites sondes.

3. Composition comprenant un réseau de sondes oligonucléotidiques selon les revendications 1-2 dans laquelle ledit fluorophore est fixé à un nucléotide de la seconde région flanquante de chacune desdites sondes et dans laquelle ledit agent d'extinction est fixé à un nucléotide de la séquence d'ADN spécifique d'intérêt de chacune desdites sondes.

4. Composition comprenant un réseau de sondes oligonucléotidiques selon les revendications 1-3 dans laquelle ledit fluorophore est un dérivé de fluorescéine.

5. Composition comprenant un réseau de sondes oligonucléotidiques selon les revendications 1-4 dans laquelle ledit agent d'extinction est un agent d'extinction Iowa Black FQ.

6. Composition comprenant un réseau de sondes oligonucléotidiques selon les revendications 1-5 dans laquelle chacune desdites sondes contient plus d'un ribonucléotide.

7. Composition comprenant un réseau de sondes oligonucléotidiques selon les revendications 1-6 dans laquelle lesdits nucléotides sont des analogues d'acides nucléiques.

8. Composition comprenant un réseau de sondes oligonucléotidiques selon les revendications 1-7 dans laquelle chacune desdites sondes est immobilisée sur un support.

9. Procédé de génotypage de répétitions en tandem courtes dans un échantillon comprenant les étapes de :
- fourniture d'un échantillon comprenant un ADN,
- amplification d'ADN dans ledit échantillon qui comprend une séquence d'ADN spécifique d'intérêt afin d'obtenir des séquences d'ADN simple brin amplifiées,
- ajout d'un réseau de sondes selon les revendications 1-8 auxdites séquences d'ADN pour obtenir des duplex de séquences d'ADN simple brin annelées auxdites sondes,
- ajout de l'enzyme RNase H2,
- chauffage du mélange de l'échantillon, de la sonde et de l'enzyme RNase H2 jusqu'à une température à laquelle l'enzyme RNase H2 est activée,
- mesure de la fluorescence lors du refroidissement dudit mélange après activation de l'enzyme RNase H2, dans lequel l'augmentation de l'intensité de fluorescence fournit des informations sur la présence ou non d'une répétition en tandem courte spécifique, complètement complémentaire, dans ledit échantillon.

10. Procédé de génotypage selon la revendication 9, dans lequel ladite amplification dans ledit échantillon est entreprise par une PCR asymétrique afin d'obtenir des séquences d'ADN simple brin amplifiées.

11. Procédé de génotypage selon les revendications 9-10 dans lequel ladite amplification dans ledit échantillon est entreprise par une PCR symétrique en utilisant des amorces marquées par la biotine ou une digestion subséquente par une exonucléase lambda afin d'obtenir des séquences d'ADN simple brin amplifiées.

12. Procédé de génotypage selon les revendications 9-11 dans lequel chacune desdites sondes est ajoutée en solution, ou est immobilisée sur un support.
